# EUROPÄISCHE PATENTSCHRIFT

(11) **EP 2 133 042 B1**
(45) Veröffentlichungstag und Bekanntmachung des Hinweises auf die Patenterteilung: **20.10.2010**
(21) Anmeldenummer: 09007092.1
(22) Anmeldetag: 27.05.2009
(51) Int. Cl.: A61F 2/52

(54) **Brustprothese**
Breast prosthesis
Prothèse de poitrine

(30) Priorität: 09.06.2008 DE 202008007695 U
(43) Veröffentlichungstag der Anmeldung: 16.12.2009
(73) Patentinhaber: Amoena Medizin-Orthopädie-Technik GmbH, 83064 Raubling (DE)
(72) Erfinder: Muscat, Dirk, Prof. Dr., 85521 Ottobrunn (DE); Moser, Stefan, 83131 Nussdorf am Inn (DE); Reusch, Michaela, 83395 Freilassing (DE)
(74) Vertreter: Laufhütte, Dieter

(56) Entgegenhaltungen:
- EP-A- 1 598 035
- DE-U1- 29 516 281

## Beschreibung

Die Erfindung betrifft eine Brustprothese nach dem Oberbegriff des Anspruchs 1. Das Dokument EP 1 598 035 A stellt der nächste Stand der Technik dar.

Es sind bereits Zweikammerbrustprothesen bekannt. Bei diesen besteht die erste Kammer aus einem in Kunststoffolien eingeschweißten, der Brustform nachgebildeten schalenförmigen Körper, der vorzugsweise aus einer additionsvernetzenden Zweikomponenten-Silikonkautschuk-Masse besteht. Weiterhin ist eine zweite Kammer vorgesehen, die der Trägerin während des Tragens zugewandt ist und sich an die erste Kammer anschließt. Diese Kammer ist üblicherweise mit einem anderen Material gefüllt als die erste Kammer.

Bereits aus der EP 0 768 068 B ist eine zweischichtige Prothese bekannt, die insbesondere auch der Versorgung von Patienten nach einer Teilamputation der Brust dient. Eine solche Prothese enthält in der dem Körper zugewandten Seite ein thixotropes Material, vorzugsweise ein pastöses Material, das im wesentlichen aus einer Mischung aus nicht vernetztem Silikonöl mit Kieselsäure besteht. Dabei können der Mischung Leichtfüllstoffe beigemengt sein. Derartige Brustprothesen neigen jedoch beim längeren Tragen zur Bildung von Sauerstoff- und Dampfblasen. Des weiteren ist die eingestellte Fließgrenze aufgrund der Feuchtigkeit nicht langzeitstabil. Daneben führt aber die Einstellung einer hohen Fließgrenze zu einem sich steif anfühlenden Material.

Aufgabe der vorliegenden Erfindung ist es, eine zweischichtige Prothese zu schaffen, bei der die zweite, dem Körper zugewandte Kammer eine dauerhaft stabile, hohe Fließgrenze kombiniert mit einer weichen Haptik aufweist. Des weiteren soll die Blasenbildung auch über einen längeren Zeitraum wirksam unterbunden werden.

Die Lösung dieser Aufgabe ergibt sich aus der Kombination der Merkmale des Anspruchs 1. Demnach wird ausgehend von einer gattungsgemäßen Brustprothese das in die zweite Kammer eingefüllte Material aus einer Flüssigkeit und einem Verdicker gebildet.

Bevorzugte Ausgestaltungen der Erfindung ergeben sich aus den sich an den Hauptanspruch anschließenden Unteransprüchen.

Vorteilhaft kann die erfindungsgemäße Lösung eine zweischichtige Prothese aufweisen, bei der die zweite, dem Körper zugewandte Kammer mit einem nicht vernetzten Gel gefüllt ist, das 20 - 95 Gew. % medizinisches Weißöl, 0,1 - 70 Gew. % Verdicker und 0 - 70 Gew.% Füllstoffe, vorzugsweise Kunststoffhohlkugeln enthält.

Als bevorzugte Verdicker können vorteilhaft Alkylsilikonwachse und/oder in Weißöl lösliche Hochpolymeren gewählt werden. Besonders bevorzugte in Weißöl lösliche Hochpolymere sind Styrol-Ethylen-Butadien-Styrol-Blockcopolymere (SEBS). Besonders bevorzugte Alkylsilikonwachse sind Silikonwachse mit langen Alkylketten. Dabei sind unter langkettigen Alkylketten vorzugsweise Ketten mit 26 bis 28 C-Atomen zu verstehen. Ein Beispiel für eine derartige lange Alkylkette ist das Produkt Belsil^{®} der Firma Wacker.

Besonders bevorzugt ist ein pastöses Material, das 20 - 95 Gew. % medizinisches Weißöl als Flüssigkeit, 1 - 7 Gew.% eines in Weißöl löslichen Hochpolymeren als Verdicker und/oder 3 - 40 Gew.% eines langkettigen Alkylsilikonwachses sowie 1 - 60 Gew.% Füllstoffe, vorzugsweise Kunststoffhohlkugeln, beinhaltet.

Ein Teil der Kunststoffhohlkugeln kann durch andere Kugeln ersetzt werden, beispielsweise durch Lurapret^{®}-Kugeln, die ein Wachs enthalten, das etwas unter der Körpertemperatur schmilzt und als Phase Change Material (PCM) bekannt ist. Diese wachsgefüllten Kugeln dienen der Klimaregulierung im Kontaktbereich Brustprothese und Körper. Des weiteren können Additive zur Farbgebung und/oder Stabilisatoren nach einer bevorzugten weiteren Ausgestaltung der Erfindung hinzugefügt werden.

Die besonders vorteilhafte Haptik der erfindungsgemäßen Brustprothesen beruht vermutlich auf der Tatsache, dass das Silikonwachs mit langen Alkylketten mikrokristalline Bereiche ausbildet, die leicht gegeneinander verschiebbar sind. So wird die "samtene" Haptik kombiniert mit einer hohen Fließgrenze erzielt.

Im folgenden werden verschiedene Ausführungsbeispiele, in denen die erfindungsgemäße Lösung manifestiert ist, wiedergegeben.

### Beispiel 1:

Gemäß dem ersten Ausführungsbeispiel setzen sich 100g Gesamtmasse des pastösen Materials wie folgt zusammen:

| | |
|---|---|
| 47,5 g | Glashohlkugeln iM30K der Firma Omya; |
| 50,4 g | medizinisches Weißöl mit einem Pourpoint von -9°C; |
| 2,1 g | TPE (Kraiburg TPE TFOSTL-B102), das auf Styrol-Ethylen-Butadien-Styrol-Blockcopolymeren (SEBS) basiert. |

### Beispiel 2:

| | |
|---|---|
| 100 g | Gesamtmasse eines pastösen Material setzt sich folgenden Bestandteilen zusammen: |
| 40 g | Glashohlkugeln der Sorte iM30K der Firma Omya; |
| 48 g | medizinisches Weißöl mit einem Pourpoint von -9°C; |
| 12 g | Belsil CM 7026VP der Firma Wacker, ein Silikonwachs mit langen Alkylketten und einem Schmelzpunkt von ca. 70 ± 5°C. |

### Beispiel 3:

Gemäß dem dritten Beispiel setzen sich 100 g Gesamtmasse des pastösen Materials gemäß der vorliegenden Erfindung zusammen aus:

| | |
|---|---|
| 63,54 g | medizinisches Weißöl mit einem Pourpoint von -9°C; |
| 4,74 g | Belsil CM 7026VP der Firma Wacker, ein Silikonwachs mit langen Alkylketten und einem Schmelzpunkt von ca. 70 ± 5°C; |
| 2,84 g | Expencels der Firma Akzo; |
| 23,71 g | Lurapret-Kugeln; |
| 5,17 g | Farbe. |

## Patentansprüche

1. Brustprothese im wesentlichen bestehend aus einem eine erste Kammer bildenden in Kunststoffolien eingeschweißten, der Brustform nachgebildeten schalenförmigen Körper, vorzugsweise aus einer additionsvernetzenden Zweikomponenten-Siliconkautschuk-Masse, und einer einer Trägerin während des Tragens zugewandten und sich an die erste Kammer anschließenden zweiten Kammer, die mit einem anderen Material gefüllt ist als die erste Kammer,
**dadurch gekennzeichnet,**
**dass** das in die zweite Kammer eingefüllte Material aus einer Flüssigkeit und einem Verdicker besteht.

2. Brustprothese nach Anspruch 1, **dadurch gekennzeichnet, dass** in der zweiten Kammer zusätzlich Füllstoffe enthalten sind.

3. Brustprothese nach Anspruch 1 oder 2, **dadurch gekennzeichnet, dass** das in die zweite Kammer eingefüllte Material 20 - 95 Gew. % Weißöl, 0,1 - 70 Gew. % Verdicker und 0 - 70 Gew.% Füllstoffe enthält.

4. Brustprothese nach Anspruch 3, **dadurch gekennzeichnet, dass** als Füllstoffe Kunststoffhohlkugeln verwendet werden.

5. Brustprothese nach einem der Ansprüche 1 bis 4, **dadurch gekennzeichnet, dass** als der Verdicker aus Alkylsilikonwachsen und/oder in Weißöl löslichen Hochpolymeren besteht.

6. Brustprothese nach Anspruch 5, **dadurch gekennzeichnet, dass** die Alkylsilikonwachse langkettig sind.

7. Brustprothese nach Anspruch 5 oder 6, **dadurch gekennzeichnet, dass** das in Weißöl lösliche Hochpolymer ein Styrol-Ethylen-Butadien-Styrol-Blockcopolymer (SEBS) ist.

8. Brustprothese nach einem der Ansprüche 1 bis 7, **dadurch gekennzeichnet, dass** das in die zweite Kammer eingefüllte Material 20-95 Gew. % medizinisches Weißöl, 1-7 Gew.% eines in Weißöl löslichen Hochpolymeren als Verdicker und/oder 10-40 Gew.% eines langkettigen Alkylsilikonwachses sowie 1-60 Gew.% Füllstoffe enthält.

9. Brustprothese nach einem der Ansprüche 1 bis 8, **dadurch gekennzeichnet, dass** das in die zweite Kammer eingefüllte Material pastös ist.

10. Brustprothese nach einem der Ansprüche 1 bis 8, **dadurch gekennzeichnet, dass** das in die zweite Kammer eingefüllte Material Additive zur Farbgebung und/oder Stabilisierung enthält.

11. Brustprothese nach einem der Ansprüche 1 bis 10, **dadurch gekennzeichnet, dass** die Füllstoffe wachsgefüllte Kugeln enthält, deren Wachsfüllung kurz unter der Körpertemperatur schmelzen.

12. Brustprothese nach einem der Ansprüche 1 bis 11, **dadurch gekennzeichnet, dass** sie als Teilprothese ausgeführt ist.

## Claims

1. Breast prosthesis consisting essentially of a shell-shaped body which forms a first chamber, is bonded into synthetic material sheets, is modelled on the shape of the breast and is preferably formed from an addition cross-linking two-component silicone rubber mass, and of a second chamber which faces the wearer when the prosthesis is being worn, adjoins the first chamber and is filled with a different material than the first chamber
**characterised in that**
the material filling the second chamber consists of a liquid and a thickener.

2. Breast prosthesis as claimed in claim 1, **characterised in that** the second chamber contains additional fillers.

3. Breast prosthesis as claimed in claim 1 or 2, **characterised in that** the material filling the second chamber contains 20 - 95% by weight of white oil, 0.1 - 70% by weight of thickener and 0 - 70% by weight of fillers.

4. Breast prosthesis as claimed in claim 3, **characterised in that** hollow synthetic material beads are used as the fillers.

5. Breast prosthesis as claimed in any one of claims 1 to 4, **characterised in that** as *[sic]* the thickener consists of alkyl silicone waxes and/or high polymers which are soluble in white oil.

6. Breast prosthesis as claimed in claim 5, **characterised in that** the alkyl silicone waxes are long-chain waxes.

7. Breast prosthesis as claimed in claim 5 or 6, **characterised in that** the high polymer which is soluble in white oil is a styrene ethylene butadiene styrene block copolymer (SEBS).

8. Breast prosthesis as claimed in any one of claims 1 to 7, **characterised in that** the material filling the second chamber contains 20 - 95% by weight of medical white oil, 1 - 7% by weight of a high polymer which is soluble in white oil as the thickener and/or 10 - 40 % by weight of a long-chain alkyl silicone wax as well as 1 - 60% by weight of fillers.

9. Breast prosthesis as claimed in any one of claims 1 to 8, **characterised in that** the material filling the second chamber is pasty.

10. Breast prosthesis as claimed in any one of claims 1 to 8, **characterised in that** the material filling the second chamber contains additives to impart colour and/or for stabilisation.

11. Breast prosthesis as claimed in any one of claims 1 to 10, **characterised in that** the fillers contains *[sic]* wax-filled beads, the wax filling of which melts just below body temperature.

12. Breast prosthesis as claimed in any one of claims 1 to 11, **characterised in that** it is formed as a partial prosthesis.

## Revendications

1. Prothèse mammaire constituée essentiellement d'un corps en forme de coque, formant une première chambre, soudé dans des feuilles de matériau synthétique, imitant la forme du sein, de préférence en une masse de caoutchouc silicone à deux composants réticulés par addition, et d'une deuxième chambre orientée vers la personne qui la porte et faisant suite à la première chambre, qui est remplie d'un autre matériau que la première chambre,
**caractérisée en ce que** le matériau introduit dans la deuxième chambre est constitué d'un liquide et d'un épaississant.

2. Prothèse mammaire selon la revendication 1, **caractérisée en ce que** la deuxième chambre contient de plus des matières de remplissage.

3. Prothèse mammaire selon la revendication 1 ou 2, **caractérisée en ce que** le matériau introduit dans la deuxième chambre contient 20-95% en poids d'huile blanche, 0,1-70% en poids d'épaississant et 0-70% en poids de matières de remplissage.

4. Prothèse mammaire selon la revendication 3, **caractérisée en ce que** comme matières de remplissage, des billes creuses en matériau synthétique sont utilisées.

5. Prothèse mammaire selon l'une des revendications 1 à 4, **caractérisée en ce que** l'épaississant est constitué de cires d'alkyle silicone et/ou de hauts polymères solubles dans l'huile blanche.

6. Prothèse mammaire selon la revendication 5, **caractérisée en ce que** les cires d'alkyle silicone sont à chaîne longue.

7. Prothèse mammaire selon la revendication 5 ou 6, **caractérisée en ce que** le haut polymère soluble dans l'huile blanche est un copolymère bloc de styrène-éthylène-butadiène-styrène (SEBS).

8. Prothèse mammaire selon l'une des revendications 1 à 7, **caractérisée en ce que** le matériau introduit dans la deuxième chambre contient 20-95% en poids d'huile blanche médicinale, 1-7% en poids d'un haut polymère soluble dans l'huile blanche comme épaississant et/ou 10-40% en poids d'une cire d'alkyle silicone à chaîne longue ainsi que 1-60% en poids de matières de charge.

9. Prothèse mammaire selon l'une des revendications 1 à 8, **caractérisée en ce que** le matériau introduit dans la deuxième chambre est pâteux.

10. Prothèse mammaire selon l'une des revendications 1 à 8, **caractérisée en ce que** le matériau introduit dans la deuxième chambre contient des additifs pour la coloration et/ou la stabilisation.

11. Prothèse mammaire selon l'une des revendications 1 à 10, **caractérisée en ce que** les matières de charge contiennent des billes remplies de cire dont le remplissage en cire fond légèrement en dessous de la température corporelle.

12. Prothèse mammaire selon l'une des revendications 1 à 11, **caractérisée en ce qu'**elle est réalisée comme prothèse partielle.
